# EUROPEAN PATENT APPLICATION

(11) **EP 4 610 987 A1**
(43) Date of publication of application: **03.09.2025**
(21) Application number: 23883092.1
(22) Date of filing: 25.10.2023
(51) Int. Cl.: G16B 50/00, G16B 25/20, G16B 30/10

(54) **METHOD AND APPARATUS FOR MANAGING APPLICATION FOR MOLECULAR DIAGNOSIS**

(30) Priority: 25.10.2022 KR 20220138530
(71) Applicant: Seegene, Inc., Seoul 05548 (KR)
(72) Inventor: SONG, Chang Hee, Seoul 05548 (KR); JOO, Weon Woo, Seoul 05548 (KR); LEE, Hyeong Sub, Seoul 05548 (KR); CHOI, Gyu Su, Seoul 05548 (KR); JAEKAL, Uk, Seoul 05548 (KR)
(74) Representative: Viering, Jentschura & Partner mbB Patent- und Rechtsanwälte
(86) International application number: PCT/KR2023/016640
(87) International publication number: WO 2024/090987

(57) **Abstract**

There is provided a method for managing an application for molecular diagnosis to be performed by a computer apparatus, the method comprising: displaying a name list of applications for molecular diagnosis that is to be executed on the computer apparatus; displaying parameter information used for processing and/or analyzing target nucleic acid amplification data in operation of the application for molecular diagnosis; receiving an application selection input to execute one of the displayed applications; receiving a parameter selection input for the displayed parameter information; and controlling the selected parameter information to be used in operation of the selected application for molecular diagnosis.

## Description

### [Technical Field]

The disclosure relates to a method and an apparatus for managing an application for molecular diagnosis.

### [Background Art]

Molecular diagnosis is a technique used to identify and analyze nucleic acids or proteins at the molecular level. Known examples of detailed technologies of such molecular diagnosis include polymerase chain reaction (PCR), isothermal nucleic acid amplification technology (INAAT), DNA sequencing and next-generation sequencing (NGS), in situ hybridization (ISH), and DNA microarray.

In each of these technologies, multiple steps are performed in order to identify and analyze a target substance. For example, a PCR test may be performed through test steps such as nucleic acid extraction, PCR setup, amplification reaction, and reaction analysis. In this case, various devices are used to perform each step, and each device may operate under the control of an application for molecular diagnosis, which is a software tool implemented for device control and analysis of generated data.

Such applications for molecular diagnosis need to manage accessible applications and additional information (i.e., information used in the application) according to the diagnostic laboratory and diagnostic performer, in order to obtain regulatory approval in accordance with country-specific standards, enhance cybersecurity, and enable efficient operation.

### [Disclosure]

### [Technical Problem]

The problem to be solved in an embodiment includes providing a technology for managing the above-described application for molecular diagnosis, but is not limited thereto.

### [Technical Solution]

In accordance with an aspect of a method for managing an application for molecular diagnosis to be performed by a computer apparatus, the method comprising: displaying a name list of applications for molecular diagnosis that is to be executed on the computer apparatus; displaying parameter information used for processing and/or analyzing target nucleic acid amplification data in operation of the application for molecular diagnosis; receiving an application selection input to execute one of the displayed applications; receiving a parameter selection input for the displayed parameter information; and controlling the selected parameter information to be used in operation of the selected application for molecular diagnosis.

Wherein the application for molecular diagnosis may include at least one application selected from a group consisting of an application used in a preparation process of molecular diagnosis, an application used for processing and/or analyzing the target nucleic acid amplification data, an application used for development of a molecular diagnostic reagent, and a combination thereof.

Wherein a memory of the computer apparatus may store a location of an executable file for the application for molecular diagnosis, the method further comprising acquiring the stored location from the memory, and wherein execution of the application for which the application selection input may be received for molecular diagnosis may be performed by the executable file stored at the stored location.

The method may further comprise acquiring access permission information of a user of the computer apparatus for the selected application for molecular diagnosis from the memory, wherein whether the selected application for molecular diagnosis is executed by the executable file may be determined depending on the acquired access permission information.

Wherein the computer apparatus may execute a plurality of applications for molecular diagnosis, and wherein the displaying the name of the application for molecular diagnosis may include displaying an application list including names of at least some of the plurality of applications for molecular diagnosis.

Wherein the application list may be configured such that a composition of applications included in the application list changes based on access permission information of a user of the computer apparatus for each of the plurality of applications included in the application list.

The method may further comprise acquiring access permission information of a user of the computer apparatus for each of the plurality of applications displayed in the application list from a memory of the computer apparatus; and allowing or blocking an operation of the selected application for molecular diagnosis depending on the acquired access permission information.

Wherein the application list may be updated each time an application for molecular diagnosis is newly installed or deleted from the computer apparatus.

Wherein the update may be configured such that an update is performed by storing or deleting a location of an executable file of the installed or deleted application for molecular diagnosis in a memory of the computer apparatus.

Wherein version information of the applications for molecular diagnosis installed in the computer apparatus may be listed in the application list.

Wherein the version information may be updated each time the application for molecular diagnosis installed in the computer apparatus is updated.

Wherein the parameter information may include at least one information of parameter information selected from a group consisting of parameter information for controlling molecular diagnosis equipment, parameter information used for processing and/or analyzing amplification data, and a combination thereof.

The method may further comprise acquiring access permission information of a user of the computer apparatus for the displayed parameter from a memory of the computer apparatus; and allowing or blocking a use of the selected parameter depending on the acquired access permission information.

Wherein a memory of the computer apparatus may store a plurality of parameter information, and wherein the method may further comprise displaying a parameter list in which a part of each of the plurality of parameter information is displayed.

Wherein the parameter list may be configured such that a composition of parameters included in the parameter list changes based on access permission information of a user of the computer apparatus for each of the parameter information included in the parameter list.

Wherein the parameter list may be updated each time a parameter is newly installed or deleted from the computer apparatus.

Wherein version information of parameters installed in the computer apparatus may be listed in the parameter list.

Wherein the version information may be updated each time the parameter installed in the computer apparatus is updated.

The method may further comprise storing, in a memory, an execution history of the application for molecular diagnosis or a usage history of the parameter information.

Wherein the execution history and/or the usage history may be stored for each user of the computer apparatus.

In accordance with an aspect of a non-transitory computer-readable storage medium including a computer program, wherein the computer program may be programmed to perform each of the steps included in the aforementioned method.

In accordance with an aspect of a computer program stored in a non-transitory computer-readable storage medium, wherein the computer program may be programmed to perform each of the steps included in the aforementioned method.

In accordance with an aspect of a computer apparatus, comprising: a memory storing at least one instruction; and a processor, wherein the at least one instruction, when executed by the processor, cause the computer apparatus to: display a name list of applications for molecular diagnosis that is to be executed on the computer apparatus; display parameter information used for processing and/or analyzing target nucleic acid amplification data in operation of the application for molecular diagnosis; receive an application selection input to execute one of the displayed applications; receive a parameter selection input for the displayed parameter information; and control the parameter information to be used in operation of the selected application for molecular diagnosis.

### [Advantageous Effects]

According to a computer apparatus in an embodiment, various effects to be described below may be achieved.

First, a user of the computer apparatus may identify what types of applications for molecular diagnosis are installed on the computer apparatus through a management module included therein.

Second, the user of the computer apparatus may execute each of the applications for molecular diagnosis through the management module included therein. Depending on embodiments, the above-described application for molecular diagnosis may not be executed without going through such management module.

Third, the user of the computer apparatus may manage the applications for molecular diagnosis through the management module. For example, installation of new applications for molecular diagnosis, updates, or deletions, etc. may be performed through the management module. In addition, management history, i.e., when a new application was installed, when and what updates were made, or when and which application was deleted, may be identified through the management module.

Fourth, exclusion of users who do not have permission to use the applications for molecular diagnosis is performed by the management module. For example, usage exclusion for all types of applications for molecular diagnosis installed on the computer apparatus, or for some of applications for molecular diagnosis, may be managed or controlled to suit each user.

### [Description of Drawings]

FIG. 1 exemplarily illustrates a concept in which a computer apparatus according to an embodiment is operated in connection with a preparation apparatus and/or an analysis apparatus.
FIG. 2 is an exemplary block diagram of the computer apparatus according to an embodiment.
FIG. 3 is an exemplary block diagram of the memory according to an embodiment.
FIG. 4 is an exemplary block diagram of a user interface for managing applications for molecular diagnosis, according to an embodiment.
FIG. 5 conceptually illustrates a configuration in which two databases included in the management module are operated in connection with the storage units according to an embodiment.
FIG. 6 conceptually illustrates execution of an application through the management module according to an embodiment.
FIG. 7 is a flowchart illustrating a method of managing applications for molecular diagnosis according to an embodiment.

### [Mode for Disclosure]

The advantages and features of the embodiments and the methods of accomplishing the embodiments will be clearly understood from the following description taken in conjunction with the accompanying drawings. However, embodiments are not limited to those embodiments described, as embodiments may be implemented in various forms. It should be noted that the present embodiments are provided to make a full disclosure and also to allow those skilled in the art to know the full range of the embodiments. Therefore, the embodiments are to be defined only by the scope of the appended claims.

In describing the embodiments of the present invention, detailed descriptions of well-known functions or configurations will be omitted if it is determined that they may unnecessarily obscure the gist of the present invention. In addition, in certain cases, there are terms arbitrarily selected by the applicant, and in this case, the meaning of the terms will be described in detail in the description of the corresponding invention. Therefore, the terms used in the present disclosure should be defined based on the meaning of the terms and the overall contents of the present disclosure, not just the name of the terms.

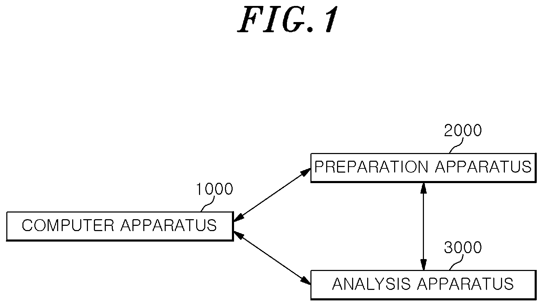

Before describing FIG. 1, the terms used herein will first be described.

The term "sample" refers to a material that includes or is presumed to include an analyte (the analyte will be described below).

Such sample may include a biological sample (e.g., cells, tissues, and body fluids from a biological source) or a non-biological sample (e.g., food, water, and soil).

Examples of biological samples may include viruses, bacteria, tissues, cells, blood (including whole blood, plasma, and serum), lymph, bone marrow fluid, sputum, swabs, aspirations, bronchial wash, bronchoalveolar lavage, nasal wash, milk, urine, feces, ocular fluid, saliva, semen, brain extract, cerebrospinal fluid (CSF), synovial fluid, appendix, spleen and tonsil tissue extracts, amniotic fluid, or ascites. In addition, naturally occurring or synthetic nucleic acid molecules isolated from a biological source may also be included as examples of the biological sample. However, the biological samples are not limited to the aforementioned examples.

The aforementioned sample may include substances used for preservation, processing, or detection, etc. of the corresponding sample. For example, the sample may include additional substances such as amplification reagents, detection reagents, preservatives, water, deionized water, saline, pH buffer, acidic solution, or basic solution. Here, the term "sample" may refer to not only a sample without the aforementioned additional substances but also a sample in which such additional substances are included.

Meanwhile, the sample described above may be referred to in various ways depending on the stage of molecular diagnosis. For example, the term "raw sample" may refer to a sample before it is processed by a first device, which will be described below. In addition, the term "analysis sample" may refer to intermediates that include an analyte and are produced during various stages of processing the sample for analysis.

Meanwhile, the above-described term "sample" may be used interchangeably with the term "specimen." Here, the term "specimen" itself refers to a target to be analyzed. More specifically, the term "specimen" may include sputum, blood, urine, stool, and the like.

Hereinafter, various embodiments of the disclosure will be described with reference to the drawings.

FIG. 1 exemplarily illustrates a concept in which a computer apparatus 1000 according to an embodiment is operated in connection with a preparation apparatus 2000 and/or an analysis apparatus 3000. However, FIG. 1 is merely illustrative.

First, in the preparation apparatus 2000 illustrated in FIG. 1, a process for preparing an analysis sample, which includes or is presumed to include an analyte, is performed.

The process of preparing the analysis sample may include a nucleic acid extraction process, a reaction solution preparation process for amplification (e.g., a reaction solution for polymerase chain reaction (PCR)), or a nucleic acid extraction and amplification reaction solution preparation process combined thereof.

Next, in the analysis apparatus 3000, a qualitative or quantitative analysis process of the analyte is performed. The sample analysis in this context includes detecting the presence of the analyte and measuring its content.

In the analysis apparatus 3000, a nucleic acid having a specific nucleotide sequence may be amplified and the amplified nucleic acid may be detected. Depending on embodiments, the analysis apparatus 3000 may include a nucleic acid amplifier that performs a nucleic acid amplification reaction through temperature control. A representative example of the nucleic acid amplifier includes a thermal cycler.

In addition, in the analysis apparatus 3000, nucleic acids may be amplified using various methods, including ligase chain reaction (LCR; see Wiedmann M, et al., "Ligase chain reaction (LCR) - overview and applications," PCR Methods and Applications, 1994 Feb; 3(4): S51-64), gap filling LCR (GLCR; see WO 90/01069, European Patent No. 439182, and WO 93/00447), Q-beta replicase amplification (Q-beta; see Cahill P, et al., Clin Chem., 37(9): 1482-5 (1991), U.S. Patent No. 5556751), strand displacement amplification (SDA; see G. T. Walker et al., Nucleic Acids Res., 20(7): 1691-1696 (1992), European Patent No. 497272), nucleic acid sequence-based amplification (NASBA; see Compton, J., Nature, 350(6313): 912 (1991)), transcription-mediated amplification (TMA; see Hofmann W. P. et al., J Clin Virol., 32(4): 289-93 (2005), U.S. Patent No. 5888779), or rolling circle amplification (RCA; see Hutchison C.A. et al., Proc. Natl Acad. Sci. USA., 102: 17332-17336 (2005)).

The computer apparatus 1000 is connected to the above-described preparation apparatus 2000 and/or analysis apparatus 3000. However, the molecular diagnosis device operable in connection with the computer apparatus 1000 is not limited to the preparation apparatus 2000 and the analysis apparatus 3000. For example, a different configuration not illustrated in FIG. 1 may be added to operate as a molecular diagnosis device, and at least one of the preparation apparatus 2000 or the analysis apparatus 3000 may not be connected to the computer apparatus 1000. However, in the following description, it is assumed that the computer apparatus 1000 operates in connection with the preparation apparatus 2000 and the analysis apparatus 3000.

In addition, in the computer apparatus 1000, applications for molecular diagnosis may be installed, which are used to control each of the above-described devices 2000 and 3000, or to analyze signals generated from the respective devices 2000 and 3000. For example, in the computer apparatus 1000, applications used in the preparation process of molecular diagnosis, applications used for processing and/or analyzing amplification data, and applications used for the development of molecular diagnostic reagents, etc. may be installed.

Further, in the computer apparatus 1000, a management module of the application for molecular diagnosis may be installed to manage access to various types of applications for molecular diagnosis installed therein, and to manage additional information related thereto. This management module may be utilized as follows.

First, a user of the computer apparatus 1000 may identify, through the management module, what types of applications for molecular diagnosis are installed on the computer apparatus 1000.

Second, a user of the computer apparatus 1000 may execute each of the applications for molecular diagnosis through the management module. Depending on embodiments, the applications for molecular diagnosis described above may not be executed without going through such management module.

Third, a user of the computer apparatus 1000 may manage the applications for molecular diagnosis through the management module. For example, installation of new applications for molecular diagnosis, updates, or deletions, etc. may be performed through the management module. In addition, management history, i.e., when a new application was installed, when and what was updated, or when and which application was deleted, may be identified through the management module.

Fourth, exclusion of users who do not have permission to use the applications for molecular diagnosis is performed by the management module. For example, usage exclusion for all types of applications for molecular diagnosis installed on the computer apparatus 1000, or for some of the applications for molecular diagnosis, may be managed or controlled to suit each user.

That is, according to an embodiment, various types of applications for molecular diagnosis installed on the computer apparatus 1000 may be easily managed.

Hereinafter, the computer apparatus 1000 will be described in more detail.

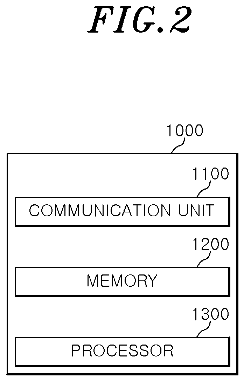

FIG. 2 is an exemplary block diagram of the computer apparatus 1000 according to an embodiment. However, FIG. 2 is merely illustrative.

Before describing FIG. 2, it should be noted that the computer apparatus 1000 according to an embodiment may be implemented in any type of user terminal and/or any type of server.

Here, the user terminal may include any type of terminal capable of interacting with a server or other computing devices. Specifically, the user terminal may include, for example, a mobile phone, a smartphone, a laptop computer, a personal digital assistant (PDA), a slate PC, a tablet PC, or an ultrabook.

Additionally, the server may include any type of computing system or computing device, such as a microprocessor, a mainframe computer, a digital processor, a portable device, or a device controller.

Reference will now be made to FIG. 2. The computer apparatus 1000 includes a communication unit 1100, a memory 1200, and a processor 1300. However, the conceptual diagram illustrated in FIG. 2 is merely illustrative. For example, the computer apparatus 1000 may further include components not illustrated in FIG. 2, or may omit at least one of the components illustrated in FIG. 2.

First, the communication unit 1100 may be configured regardless of its communication mode, whether wired or wireless, and may be composed of various communication networks such as a Personal Area Network (PAN) or a Wide Area Network (WAN). Additionally, the communication unit 1100 may operate based on the World Wide Web (WWW) and may also utilize wireless transmission technologies used for short-range communication, such as Infrared Data Association (IrDA) or Bluetooth.

The computer apparatus 1000, through the communication unit 1100, may exchange data with or control and manage the above-described preparation apparatus or analysis apparatus.

The memory 1200 may store at least one instruction executable by the processor 1300. In an embodiment, the memory 1200 may store any type of information generated or determined by the processor 1300, as well as any type of information received by the computer apparatus 1000.

In an embodiment, the memory 1200 may be a storage medium or a computer-readable recording medium that stores computer software allowing the processor 1300 to perform operations according to embodiments of the disclosure. In this case, the memory 1200 may refer to computer-readable media that store software code required to perform embodiments of the disclosure, data to be processed by the code, and results generated by the execution of the code.

Here, the software storable in the memory 1200 may be an application for molecular diagnosis or the above-described management module of the application for molecular diagnosis. A more detailed description of the management module of the application for molecular diagnosis will be provided below.

In one embodiment, the memory 1200 may refer to any type of storage medium. For example, the memory 1200 may include at least one type of storage medium, such as flash memory type, hard disk type, multimedia card micro type, card-type memory (e.g., SD or XD memory), Random Access Memory (RAM), Static Random Access Memory (SRAM), Read-Only Memory (ROM), Electrically Erasable Programmable Read-Only Memory (EEPROM), Programmable Read-Only Memory (PROM), magnetic memory, magnetic disk, or optical disk.

By executing at least one instruction stored in the memory 1200, the processor 1300 may perform the technical features according to embodiments of the present disclosure, which will be described later. In one embodiment, the processor 1300 may be composed of at least one core and may include a processor for data analysis and/or processing, such as a central processing unit (CPU), general purpose graphics processing unit (GPGPU), or tensor processing unit (TPU) of the computer apparatus 1000.

Hereinafter, a description will be given of the structure of the memory 1200 configured to perform a method of managing an application for molecular diagnosis according to an embodiment.

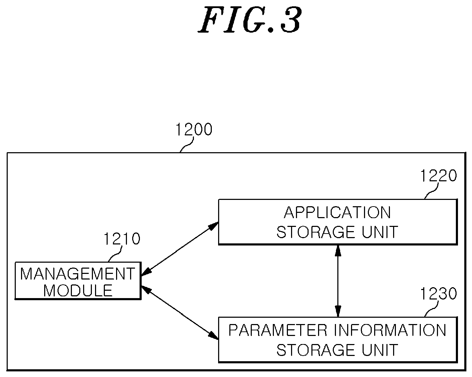

FIG. 3 is an exemplary block diagram of the memory 1200 according to an embodiment. However, FIG. 3 is merely illustrative.

With reference to FIG. 3, the memory 1200 may include a management module 1210, a molecular diagnosis application storage unit 1220, and a parameter information storage unit 1230.

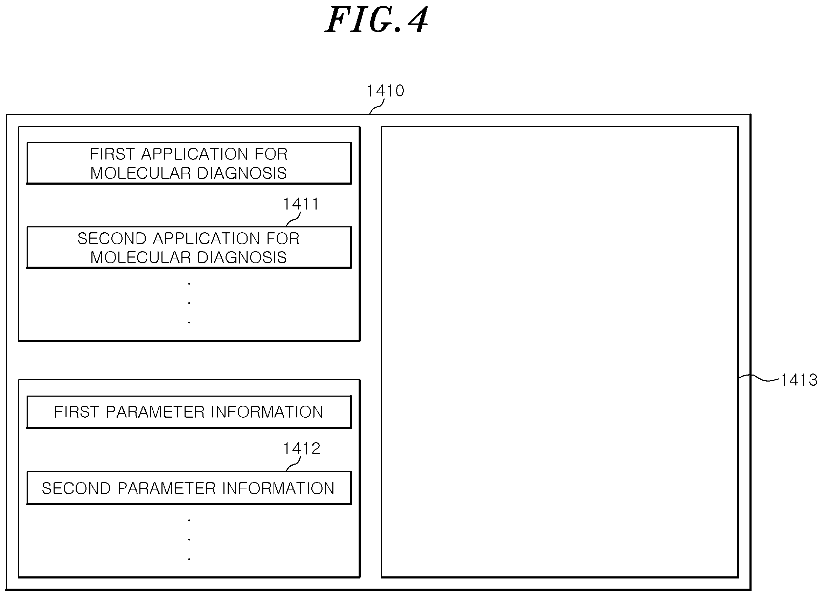

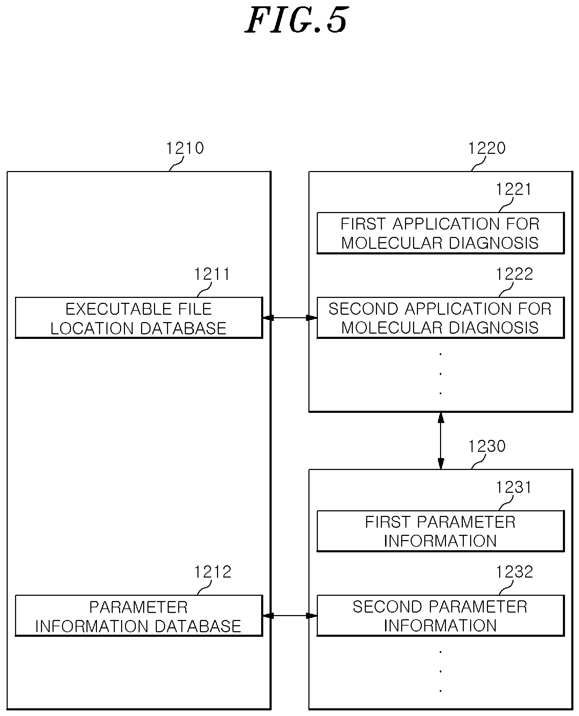

First, the management module 1210 may be an application generated to manage applications for molecular diagnosis and additional information used therewith, which are stored in the molecular diagnosis application storage unit 1220 and the parameter information storage unit 1230. When the management module 1210 according to an embodiment is executed by the processor 1300 illustrated in FIG. 2, the molecular diagnosis application storage unit 1220 and the parameter information storage unit 1230 may be managed using databases connected to each of the molecular diagnosis application storage unit 1220 and the parameter information storage unit 1230. This will be described in more detail with reference to FIGS. 4 and 5.

Next, in the molecular diagnosis application storage unit 1220, at least one application may be included, such as an application used in the preparation process of molecular diagnosis, an application used for processing and/or analyzing amplification data, or an application used for the development of molecular diagnostic reagents.

Here, the preparation of molecular diagnosis refers to the process of preparing an analysis sample, which includes or is presumed to include an analyte, and includes a nucleic acid extraction process from a specimen, a reaction solution preparation process for amplification (e.g., a reaction solution for polymerase chain reaction (PCR)), or a nucleic acid extraction and amplification reaction solution preparation process combined thereof.

In addition, the amplification data may be data generated during a process performed to detect the presence of an analyte or to measure its content. For example, when the analyte is a nucleic acid, the data generated during a process of amplifying a nucleic acid having a specific nucleotide sequence may be referred to as amplification data.

Next, the parameter information storage unit 1230 may store at least one type of parameter information, such as parameter information for controlling molecular diagnosis equipment according to each molecular diagnostic reagent, or parameter information used for processing and/or analyzing amplification data.

Here, the parameter information for controlling the molecular diagnosis equipment may be an input value or setting value that may be input or preset for the control of the molecular diagnosis equipment, including the above-described preparation apparatus 2000 and analysis apparatus 3000. For example, it may be the type of analyte to be analyzed by the molecular diagnosis equipment, the type of reagent used with the analyte, and input values, setting values, or algorithm types related to processes performed in the equipment.

In addition, in the parameter information storage unit 1230, a value functioning as a criterion for reading the presence or absence of a target analyte in a sample during a presence/absence reading process may be stored as parameter information.

Hereinafter, a description will be given of operations that may be performed by the processor 1300 in the computer apparatus 1000, by executing at least one instruction stored in the memory 1200.

FIG. 4 is an exemplary block diagram of a user interface 1410 for managing applications for molecular diagnosis, displayed on a display unit (not illustrated) of the computer apparatus 1000 according to an embodiment. However, FIG. 4 is merely illustrative.

The user interface 1410 according to an embodiment may be operated when the management module 1210 stored in the memory 1200 is executed by the processor 1300, and may be displayed on the display unit (not illustrated) provided in the computer apparatus 1000, but is not limited thereto.

The user interface 1410 may include a molecular diagnosis application selection unit 1411, a parameter information selection unit 1412, and a selection item display unit 1413. However, the configuration included in the user interface 1410 is not limited to the molecular diagnosis application selection unit 1411, the parameter information selection unit 1412, and the selection item display unit 1413. For example, other components not illustrated in FIG. 4 may be added to perform operations for managing applications for molecular diagnosis. However, in the following description, it is assumed that a molecular diagnosis application management U/I 6000 includes the molecular diagnosis application selection unit 1411, the parameter information selection unit 1412, and the selection item display unit 1413.

With reference to FIG. 4, the user interface 1410 may include the molecular diagnosis application selection unit 1411, which provides an executable application list in which the names of at least one executable application for molecular diagnosis in the computer apparatus 1000 are listed. Here, the executable application for molecular diagnosis refers to an application for molecular diagnosis that has been installed in the computer apparatus 1000 in a state that allows execution, and the storage location of the application file is not limited. Through the user interface 1410, any one of the applications for molecular diagnosis listed in the executable application list may be selected. A description regarding the listing of executable applications will be provided below with reference to the following drawings.

In addition, the user interface 1410 may include a parameter information selection unit 1412, which provides a parameter information list. The parameter information selection unit 1412 may provide a list that includes at least one parameter information including information used for processing and/or analyzing amplification data during the operation of an application for molecular diagnosis. Additionally, the parameter information selection unit 1412 may also provide the parameter information for each molecular diagnostic reagent. Further, through the user interface 1410, at least one parameter information may be selected by the parameter information selection unit 1412.

In addition, the user interface 1410 may include a selection item display unit 1413, which may display items selected by the molecular diagnosis application selection unit 1411 or the parameter information selection unit 1412. In the selection item display unit 1413, the content related to the application may be displayed, such as the operating status of the device linked with the application selected by the user and analysis content of data calculated by the device. In addition, the selection item display unit 1413 may display additional information related to the parameter information that the user selected.

In this case, the displayed parameter information may be selected and displayed only for the parameters that may be used in the application selected by the user.

As described above, through the user interface 1410, the user may identify what types of applications for molecular diagnosis are installed in the computer apparatus 1000, execute each application for molecular diagnosis, and also select parameter information applicable to each.

Meanwhile, the user interface 1410 according to an embodiment may also perform the functions related to a user access in addition to the functions related to installation verification and execution of the applications for molecular diagnosis as described above. For example, a user may not be allowed to execute the above-described application for molecular diagnosis unless the user goes through the user interface 1410 on the computer apparatus 1000. In addition, usage exclusion for users without permission to use the applications for molecular diagnosis may also be one of the functions of the user interface 1410.

Hereinafter, a description will be given of the configuration and operation examples of the management module 1210 related to the operation of the user interface for such functions of the user interface.

FIG. 5 conceptually illustrates a configuration in which two databases 1211 and 1212 included in the management module 1210 are operated in connection with the storage units 1220 and 1230, according to an embodiment. However, FIG. 5 is merely illustrative.

With reference to FIG. 5, the management module 1210 according to an embodiment includes an executable file location database 1211 and a parameter information database 1212.

First, the executable file location database 1211 will be described. The management module 1210 may receive information from the molecular diagnosis application storage unit 1220, which stores at least one application for molecular diagnosis. The information received by the management module 1210 from the molecular diagnosis application storage unit 1220 may include the location of the executable file for each of the applications for molecular diagnosis 1221 and 1222 stored in the molecular diagnosis application storage unit 1220. Here, the location of the executable file refers to the unique location, within the user's computer apparatus, of the file that may execute the application for molecular diagnosis in the user's computer apparatus. This is commonly referred to as the executable file path.

Meanwhile, the location of the executable file delivered to the management module 1210 may be stored in the executable file location database 1211 included in the management module. In addition, the executable file location database 1211 may perform updates based on a comparison between newly stored information and pre-stored information.

Next, the parameter information database 1212 will be described. The management module 1210 may receive information from the parameter information storage unit 1230, which stores at least one parameter information. The information received by the management module 1210 from the parameter information storage unit 1230 may include at least part of the parameter information, which is stored for each molecular diagnostic reagent in the parameter information storage unit 1230 and is used for processing and/or analyzing amplification data during the operation of the application for molecular diagnosis.

Meanwhile, the parameter information delivered to the management module 1210 may be stored in the parameter information database 1212 included in the management module. Additionally, the parameter information database 1212 may perform updates based on a comparison between newly stored information and pre-stored information.

Hereinafter, an example of an operation performed using the database structure of the management module 1210 will be described.

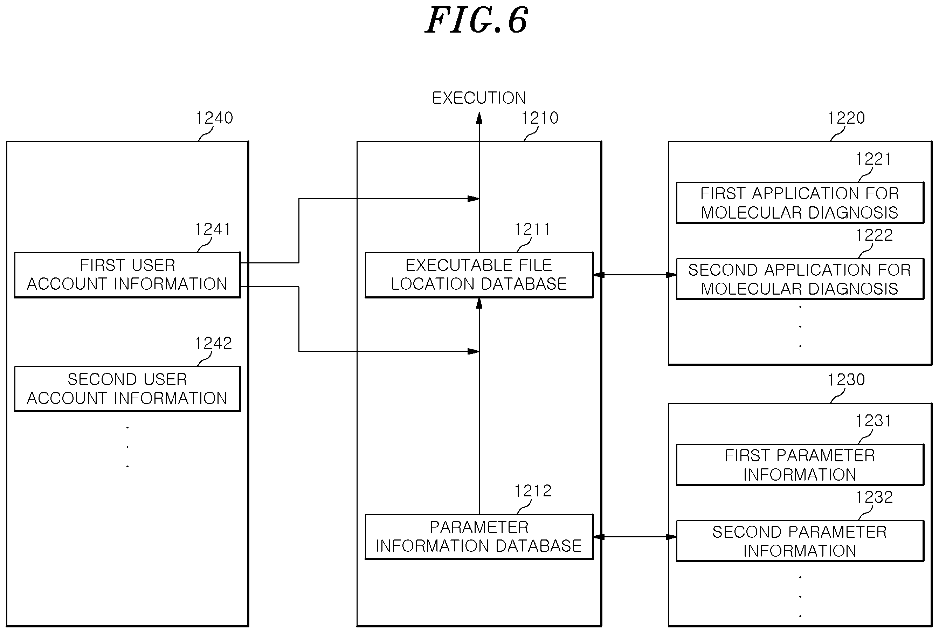

FIG. 6 conceptually illustrates execution of an application through the management module 1210 according to an embodiment. However, FIG. 6 is merely illustrative.

With reference to FIG. 6, an embodiment will be described in which an application for molecular diagnosis is executed using the information stored in the databases 1211 and 1212 of the management module 1210 described above.

The management module 1210 according to an embodiment includes the executable file location database 1211, and is thus implemented to store at least one executable file location. In this case, as only the location of the executable file, not the executable file itself, is stored, it is implemented to control the execution of the application for molecular diagnosis connected to the management module 1210. The method of storing or sharing the executable file may be performed by another configuration not illustrated in FIG. 6. However, in the following description, it is assumed that the management module 1210 stores and controls the executable file location in order to control the execution of the application for molecular diagnosis.

The management module 1210 according to an embodiment includes the executable file location database 1211 and is thus implemented to store the locations of executable files of applications for molecular diagnosis executable on the computer apparatus 1000. In this case, at least one executable application for molecular diagnosis 1221, 1222 may be stored in the molecular diagnosis application storage unit 1220, which is provided in the computer apparatus or a device connected thereto. For the executable files of the stored respective applications for molecular diagnosis, the location of the executable file, which is a unique location within the computer apparatus, may be stored in the executable file location database 1211. The management module 1210, or a user interface executed thereby, may provide the stored executable file location to provide the user with applications for molecular diagnosis that are executable. In this case, the term "executable" refers to the state in which an application for molecular diagnosis is installed on the computer apparatus in such a manner that it is executable. In this case, the management module 1210 may deliver the name of the application for molecular diagnosis for which it knows the executable file location, thereby delivering the user with the executable applications for molecular diagnosis.

With reference to FIG. 6, the management module 1210 may acquire a user account through a user account verification process. In this case, the user who may access the management module 1210 may be one of a plurality of users whose respective account information 1241, 1242 is included in a user account information group 1240. For each of at least one user, the user account may be collected during access to the management module 1210 through the user account verification process, and the collected user account may be used in executing an application for molecular diagnosis.

The management module 1210 may set access permissions per application for molecular diagnosis for each of at least one user. Additionally, the management module 1210 may collect user accounts when each user accesses the management module 1210. The management module 1210 may compare the user account that accesses the management module 1210 with the application-specific access permissions, and determine whether to allow execution of the application for molecular diagnosis. After verifying the user-specific access permissions for the application for molecular diagnosis the user attempts to execute, the management module 1210 may allow execution only when the corresponding user account has the access permission for the corresponding application.

In another embodiment related to execution based on user-specific access permissions, the management module 1210 may generate a variable including at least one item of the user-specific access permission or the user account information. The variable including the user-specific access permission and/or the user account information may be input to the computer apparatus together with the executable file location of the application for molecular diagnosis selected by the user. For example, the variable and the executable file location may be input into the operating system of the computer apparatus. The computer apparatus that receives the variables and the executable file location may execute the application only when the received variable matches the user-specific, application-specific access permissions pre-stored in the management module 1210.

The management module 1210 may execute an application for molecular diagnosis by using both user account information acquired during platform access and the executable file location of the executable file location database 1211 included in the platform. The management module 1210 may reinforce the conditions for executing the application for molecular diagnosis to enhance cybersecurity. For example, when a user accesses an executable file through a path other than the executable file location in the executable file location database 1211, that is, without going through the management module 1210, the corresponding application may not be executed. In this case, a variable including the user account information generated during access to the management module 1210 may be implemented as an essentially required method for application execution.

Through the above-described embodiments, the management module 1210 may distinguish and control the execution of each application for molecular diagnosis for each of a plurality of users. In addition, access to applications for molecular diagnosis that does not pass through the management module 1210 on the computer apparatus may be restricted. These configurations may enhance the cybersecurity performance of the applications for molecular diagnosis.

Meanwhile, the parameter information database 1212 included in the management module 1210 stores parameter information in various formats that may be used in the applications for molecular diagnosis. The parameter information may include parameter information for controlling the molecular diagnosis equipment, and parameter information used for processing and/or analyzing amplification data. Such parameter information may be stored in the parameter information database 1212 for each molecular diagnostic reagent. Similar to the applications for molecular diagnosis, the storage status of the parameter information may be provided to the user through the management module 1210. In addition, in the process of controlling the use of parameter information in the operation process of the application for molecular diagnosis, the user's access permission may be used. In this specification, the term 'execution' of an application refers to the overall process of running and operating an application that is launched and operated by the user's selection, while the term 'operation' of an application emphasizes the process of performing the application's unique functions after the application is launched.

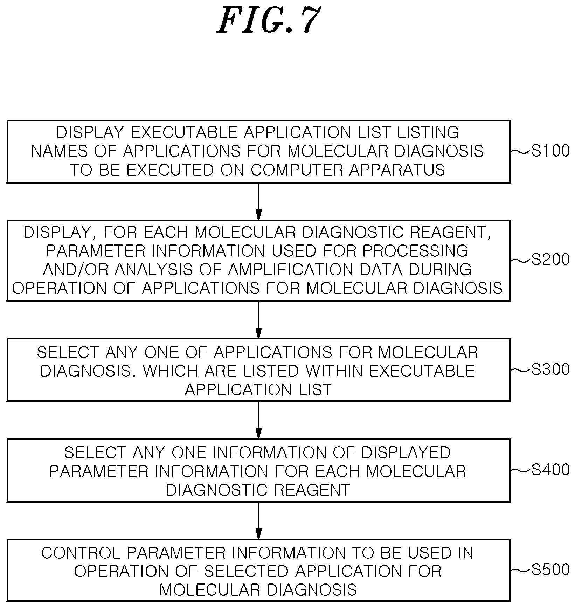

FIG. 7 is a flowchart illustrating a method of managing applications for molecular diagnosis according to an embodiment. However, FIG. 7 is merely illustrative.

Referring to FIG. 7, in accordance with an aspect of a method for managing an application for molecular diagnosis to be performed by a computer apparatus 1000, the method comprising: displaying an executable application list, which lists the names of applications for molecular diagnosis executable on the computer apparatus (S100); displaying parameter information used for processing and/or analyzing amplification data during the operation of the application for molecular diagnosis for each molecular diagnostic reagent (S200); receiving an application selection input for an application among the applications for molecular diagnosis listed in the executable application list (S300); receiving a parameter selection input for the displayed parameter information for each molecular diagnostic reagent (S400); and controlling the selected parameter information to be used in operation of the selected application for molecular diagnosis (S500).

Specifically, in step S100, an executable application list, which lists the names of applications for molecular diagnosis executable on the computer apparatus, is displayed. Here, the term executable application for molecular diagnosis refers, as previously described, to a state in which the application is installed on the computer apparatus in a manner that allows it to be executed, and the storage location of the application file is not limited.

In accordance with an aspect of a method for managing an application for molecular diagnosis, wherein the application list may be updated each time an application for molecular diagnosis is newly installed or deleted from the computer apparatus.

In accordance with an aspect of a method for managing an application for molecular diagnosis, wherein version information of the applications for molecular diagnosis installed in the computer apparatus is listed in the application list. Here, wherein the version information may be updated each time the application for molecular diagnosis installed in the computer apparatus is updated.

In accordance with an aspect of a method for managing an application for molecular diagnosis, the method may further comprise acquiring access permission information of a user of the computer apparatus for the displayed parameter from a memory of the computer apparatus; and allowing or blocking a use of the selected parameter depending on the acquired access permission information.

In accordance with an aspect of a method for managing an application for molecular diagnosis, wherein a memory of the computer apparatus may store a location of an executable file for the application for molecular diagnosis, the method may further comprise acquiring the stored location from the memory, and wherein execution of the selected application for molecular diagnosis may be performed by the executable file stored at the stored location. Here, the method may further comprise acquiring access permission information of a user of the computer apparatus for the selected application for molecular diagnosis from the memory, wherein whether the selected application for molecular diagnosis may be executed by the executable file is determined depending on the acquired access permission information.

In accordance with an aspect of a method for managing an application for molecular diagnosis, the method may further comprise storing, in a memory, an execution history of the application for molecular diagnosis or a usage history of the parameter information. Here, wherein the execution history and/or the usage history may be stored for each user of the computer apparatus.

In accordance with an aspect of a method for managing an application for molecular diagnosis, wherein the application for molecular diagnosis may include at least one application selected from a group consisting of an application used in a preparation process of molecular diagnosis, an application used for processing and/or analyzing the target nucleic acid amplification data, an application used for development of a molecular diagnostic reagent, and a combination thereof.

Next, in step S200, parameter information used for processing and/or analyzing amplification data during the operation of the application for molecular diagnosis is displayed for each molecular diagnostic reagent.

In the method of managing applications for molecular diagnosis according to an embodiment, the parameter information may be updated when a molecular diagnostic reagent is newly developed or when a previously developed molecular diagnostic reagent is modified. In this case, the parameter information may include version information, and the version information may be updated each time the parameter information is updated.

In the method of managing applications for molecular diagnosis according to an embodiment, wherein the parameter information includes at least one information of parameter information selected from a group consisting of parameter information for controlling molecular diagnosis equipment, parameter information used for processing and/or analyzing amplification data, and a combination thereof.

In step S300, one of the applications for molecular diagnosis listed in the executable application list is selected.

In step S400, one of the displayed parameter information for each molecular diagnostic reagent is selected.

In step S500, during the operation of the selected application for molecular diagnosis, the selected parameter information is controlled to be used.

Meanwhile, according to an embodiment, each step included in the above-described method may be implemented in the form of a computer program stored in a computer-readable recording medium that is programmed to include and perform the respective steps.

In addition, according to an embodiment, each step included in the above-described method may be implemented in the form of a computer-readable recording medium storing a computer program programmed to include and perform the respective steps.

The above description is merely exemplary description of the technical scope of the present disclosure, and it will be understood by those skilled in the art that various changes and modifications can be made without departing from original characteristics of the present disclosure. Therefore, the embodiments disclosed in the present disclosure are intended to explain, not to limit, the technical scope of the present disclosure, and the technical scope of the present disclosure is not limited by the embodiments. The protection scope of the present disclosure should be interpreted based on the following claims and it should be appreciated that all technical scopes included within a range equivalent thereto are included in the protection scope of the present disclosure.

## Claims

1. A method for managing an application for molecular diagnosis to be performed by a computer apparatus, the method comprising:
displaying a name list of applications for molecular diagnosis that is to be executed on the computer apparatus;
displaying parameter information used for processing and/or analyzing target nucleic acid amplification data in operation of the application for molecular diagnosis;
receiving an application selection input to execute one of the displayed applications;
receiving a parameter selection input for the displayed parameter information; and
controlling the selected parameter information to be used in operation of the selected application for molecular diagnosis.

2. The method of claim 1, wherein the application for molecular diagnosis includes at least one application selected from a group consisting of an application used in a preparation process of molecular diagnosis, an application used for processing and/or analyzing the target nucleic acid amplification data, an application used for development of a molecular diagnostic reagent, and a combination thereof.

3. The method of claim 1, wherein a memory of the computer apparatus stores a location of an executable file for the application for molecular diagnosis, the method further comprising acquiring the stored location from the memory, and
wherein execution of the selected application for molecular diagnosis is performed by the executable file stored at the stored location.

4. The method of claim 3, further comprising:
acquiring access permission information of a user of the computer apparatus for the selected application for molecular diagnosis from the memory,
wherein whether the selected application for molecular diagnosis is executed by the executable file is determined depending on the acquired access permission information.

5. The method of claim 1, wherein the computer apparatus executes a plurality of applications for molecular diagnosis, and
wherein the displaying the name of the application for molecular diagnosis includes displaying an application list including names of at least some of the plurality of applications for molecular diagnosis.

6. The method of claim 5, wherein the application list is configured such that a composition of applications included in the application list changes based on access permission information of a user of the computer apparatus for each of the plurality of applications included in the application list.

7. The method of claim 5, further comprising:
acquiring access permission information of a user of the computer apparatus for each of the plurality of applications displayed in the application list from a memory of the computer apparatus; and
allowing or blocking an operation of the selected application for molecular diagnosis depending on the acquired access permission information.

8. The method of claim 5, wherein the application list is updated each time an application for molecular diagnosis is newly installed or deleted from the computer apparatus.

9. The method of claim 8, wherein the update is configured such that an update is performed by storing or deleting a location of an executable file of the installed or deleted application for molecular diagnosis in a memory of the computer apparatus.

10. The method of claim 5, wherein version information of the applications for molecular diagnosis installed in the computer apparatus is listed in the application list.

11. The method of claim 10, wherein the version information is updated each time the application for molecular diagnosis installed in the computer apparatus is updated.

12. The method of claim 1, wherein the parameter information includes at least one information of parameter information selected from a group consisting of parameter information for controlling molecular diagnosis equipment, parameter information used for processing and/or analyzing amplification data, and a combination thereof.

13. The method of claim 1, further comprising:
acquiring access permission information of a user of the computer apparatus for the displayed parameter from a memory of the computer apparatus; and
allowing or blocking a use of the selected parameter depending on the acquired access permission information.

14. The method of claim 1, wherein a memory of the computer apparatus stores a plurality of parameter information, and
wherein the method further comprises displaying a parameter list in which a part of each of the plurality of parameter information is displayed.

15. The method of claim 14, wherein the parameter list is configured such that a composition of parameters included in the parameter list changes based on access permission information of a user of the computer apparatus for each of the parameter information included in the parameter list.

16. The method of claim 15, wherein the parameter list is updated each time a parameter is newly installed or deleted from the computer apparatus.

17. The method of claim 14, wherein version information of parameters installed in the computer apparatus is listed in the parameter list.

18. The method of claim 17, wherein the version information is updated each time the parameter installed in the computer apparatus is updated.

19. The method of claim 1, further comprising:
storing, in a memory, an execution history of the application for molecular diagnosis or a usage history of the parameter information.

20. The method of claim 19, wherein the execution history and/or the usage history is stored for each user of the computer apparatus.

21. A computer program stored on a non-transitory computer-readable storage medium that is programmed to include and perform each step included in the method according to any one of claims 1 to 20.

22. A non-transitory computer-readable storage medium storing a computer program that is programmed to include and perform each step included in the method according to any one of claims 1 to 20.

23. A computer apparatus, comprising:
a memory storing at least one instruction; and
a processor,
wherein the at least one instruction, when executed by the processor, cause the computer apparatus to:
display a name list of applications for molecular diagnosis that is to be executed on the computer apparatus;
display parameter information used for processing and/or analyzing target nucleic acid amplification data in operation of the application for molecular diagnosis;
receive an application selection input to execute one of the displayed applications;
receive a parameter selection input for the displayed parameter information; and
control the parameter information to be used in operation of the selected application for molecular diagnosis.
